# EUROPEAN PATENT APPLICATION

(11) **EP 2 145 579 A1**
(43) Date of publication of application: **20.01.2010**
(21) Application number: 09163471.7
(22) Date of filing: 23.06.2009
(51) Int. Cl.: A61B 5/00, G01N 21/75, G01N 33/50

(54) **System and Method for Optical Continuous Detection of an Analyte in Bloodstream**

(30) Priority: 27.06.2008 US 76225 P
(71) Applicant: Tyco Healthcare Group LP, Mansfield, MA 02048 (US)
(72) Inventor: Meyer, Peter, Shrewsbury, MA 01545 (US)
(74) Representative: Gray, James

(57) **Abstract**

A method for performing a blood assay includes the steps of positioning an optical biosensor in fluid communication with a blood vessel whereby blood from the blood vessel contacts the biosensor. The biosensor includes at least one material adapted to bind to an analyte. The method also includes the steps of detecting a change in at least one optical property of the biosensor resulting from binding of the at least one material with the analyte and transmitting a continuous signal representative of the change in at least one optical property of the biosensor to a display module to provide real time analysis by a clinician.

## Description

### CROSS-REFERENCE TO RELATED APPLICATION

The present application claims the benefit of U.S. Provisional Application Serial No. 61/076,225 entitled "SYSTEM AND METHOD FOR OPTICAL CONTINUOUS DETECTION OF AN ANALYTE IN BLOODSTREAM" filed on June 27, 2008 by Peter Meyer, the entire disclosure of which is incorporated by reference herein.

### BACKGROUND

### Technical Field

The present disclosure relates to a system and method for performing blood assays. In particular, the present disclosure is directed to in vivo optical biosensors configured to continuously monitor blood to detect the presence and/or concentration of an analyte of interest.

### Background of Related Art

Various types of blood analyzers for detecting specific analytes of interest (e.g., proteins) are known in the art. A conventional blood analyzer utilizes a sensor to detect the presence of the analyte and optionally determines the concentration thereof. In vitro methods are usually utilized to obtain a blood sample from a blood vessel and subsequently provide the sample to the blood analyzer for analysis.

Rapid diagnosis of a clinical condition is key to limiting severity of the illness. Conventional blood analyzers, which perform an in-vitro analysis on blood drawn from the patient at a single point in time, are appropriate when the concentration of the blood analyte of interest (e.g. viral infection) is approximately constant over the treatment time. However, known blood analyzers of the type aforementioned present a major drawback which detracts from their overall usefulness and effectiveness. In particular, the conventional blood analyzer is incapable of providing real or present time data of the analyte of interest present in the blood stream. In clinical situations where the concentration of an analyte of interest in the blood can be expected to change rapidly (e.g. myocardial infarction), conventional blood analyzers fail to detect the clinically meaningful rate of change in analyte concentration. Moreover, the conventional blood analyzer is limited in that it can only indicate the presence of the analyte at the moment when the sample of blood was drawn. In many applications, the amount of analyte present does not exhibit elevated concentrations in the bloodstream until several hours after the biological event. It is therefore possible to misdiagnose the patient because the blood used in the diagnostic assay was drawn before the analyte of interest had reached the threshold of clinical significance.

One conventional solution involves performing multiple in vitro assays to periodically screen the blood for elevated concentration of the analyte. However, performing multiple assays is overly invasive to the patient. In addition, this solution is also imperfect since there is a possibility that occurrence of the biological event may be missed, or its detection delayed by as long as the time interval between successive blood draws.

This particular problem is acutely prevalent in the field of monitoring of acute myocardial infarction patients. Biochemical markers associated with myocardial infarction (e.g., cardiac troponin) are detectable in the patient's blood stream about 3 to 8 hours from the onset of the condition. In the absence of other indications of the condition (e.g., electrocardiogram indicators, acute distress, etc.), a patient complaining of physical conditions associated with myocardial infarction (e.g., chest pain) is typically observed for up to 12 hours to rule out the infarction as the cause of the symptoms. Conventionally, cardiac marker assays are typically performed serially at 6-8 hour intervals in order to detect a recent infarction. Due to the relatively long time periods between assays, a true infarction patient with biological signs of infarction may, as a result, wait for many hours before the signs are detected. Consequently, there is a delay in providing therapy to the patient.

Therefore it would be desirable to provide a blood analyzer that continuously detects the presence of an analyte in a bloodstream to allow for instantaneous and continuous detection of elevated analyte concentration.

### SUMMARY

The present disclosure relates to a system and method for performing in vivo blood assay to detect the presence and concentration of an analyte. The system includes an optical biosensor having an antibody material adapted to bind to the analyte of interest. The biosensor is in fluid communication with a blood vessel such that blood continuously contacts the biosensor and the analyte binds to the antibody material. Excitation light is supplied to the biosensor and passes therethrough. Certain properties of the emitted light are affected by the presence of analyte at or near the surface of the biosensor due to binding of the analyte to the antibody material. Changes in the emitted light are monitored and analyzed by a detector which then calculates the concentration of the analyte in the bloodstream. The calculations are then transmitted to a monitor for display.

According to one aspect of the present disclosure, a medical analyzer to assay blood for an analyte is disclosed. The analyzer includes an optical biosensor adapted to be in fluid communication with a blood vessel whereby blood from the blood vessel contacts the biosensor. The biosensor includes at least one material adapted to bind to an analyte of the blood. The analyzer also includes an excitation source for supplying excitation light to the biosensor and a detector adapted to detect a change in emitted light by the biosensor resulting from the binding of the at least one material of the biosensor with the analyte of the blood. The detector is also adapted to generate a continuous signal representative of the change in the at least one optical property of the biosensor to provide real time analysis by a clinician.

According to another aspect of the present disclosure, a medical analyzer to assay blood for an analyte is disclosed. The analyzer includes an optical biosensor adapted to be in fluid communication with a blood vessel whereby blood from the blood vessel contacts the biosensor. The biosensor includes at least one material adapted to bind to an analyte of the blood. The biosensor is adapted to transmit a change in at least one optical property of the biosensor resulting from the binding of the at least one material of the biosensor with the analyte of the blood.

According to another aspect of the present disclosure, a medical analyzer to assay blood for an analyte is disclosed. The analyzer includes an optical biosensor adapted to be in fluid communication with a blood vessel whereby blood from the blood vessel contacts the biosensor. The biosensor includes at least one material adapted to bind to an analyte of the blood. The analyzer also includes an excitation source for supplying excitation light to the biosensor and a detector adapted to detect a change in emitted light by the biosensor resulting from the binding of the at least one material of the biosensor with the analyte of the blood. The detector is also adapted to generate signal representative of the second time derivative of the at least one optical property of the biosensor to provide a measurement of the rate of change in analyte concentration to a clinician for analysis.

A method for performing a tissue assay is also contemplated according to the present disclosure. The method includes the steps of positioning a biosensor in fluid communication with tissue of the patient whereby the tissue contacts the biosensor. The biosensor includes a material adapted to bind to an analyte. The method also includes the steps of detecting a change in at least one optical property of the biosensor resulting from binding of the at least one material with the analyte and transmitting a continuous signal representative of the change in the at least one optical property of the biosensor to a display module to provide real time analysis by a clinician.

A method for performing a blood assay is also contemplated according to the present disclosure. The method includes the steps of positioning an optical biosensor in fluid communication with a blood vessel of the patient whereby blood from the blood vessel contacts the biosensor. The biosensor includes a material adapted to bind to an analyte. The method also includes the steps of detecting a change in at least one optical property of the biosensor resulting from binding of the at least one material with the analyte and transmitting a continuous signal representative of the change in the at least one optical property of the biosensor to a display module to provide real time analysis by a clinician.

### BRIEF DESCRIPTION OF THE DRAWINGS

Various embodiments of the present disclosure are described herein with reference to the drawings wherein:
Fig. 1 is a view of a blood analyzer according to the present disclosure accessing a blood vessel;
Fig. 2 is a cross-sectional view of the probe of the blood analyzer;
Fig. 3 is a cross-sectional view of entry end of the probe of the blood analyzer illustrating the biosensor within the probe according to the present disclosure;
Figs. 4A-B are diagrams of excitation and emitted light waveforms;
Fig. 5 is a cross-sectional view of entry end of the probe of the blood analyzer illustrating the biosensor within the probe according to another embodiment of the present disclosure; and
Fig. 6 is a flow diagram of a method for performing a blood assay according to the present disclosure.

### DETAILED DESCRIPTION

Particular embodiments of the present disclosure are described hereinbelow with reference to the accompanying drawings. In the following description, well-known functions or constructions are not described in detail to avoid obscuring the present disclosure in unnecessary detail.

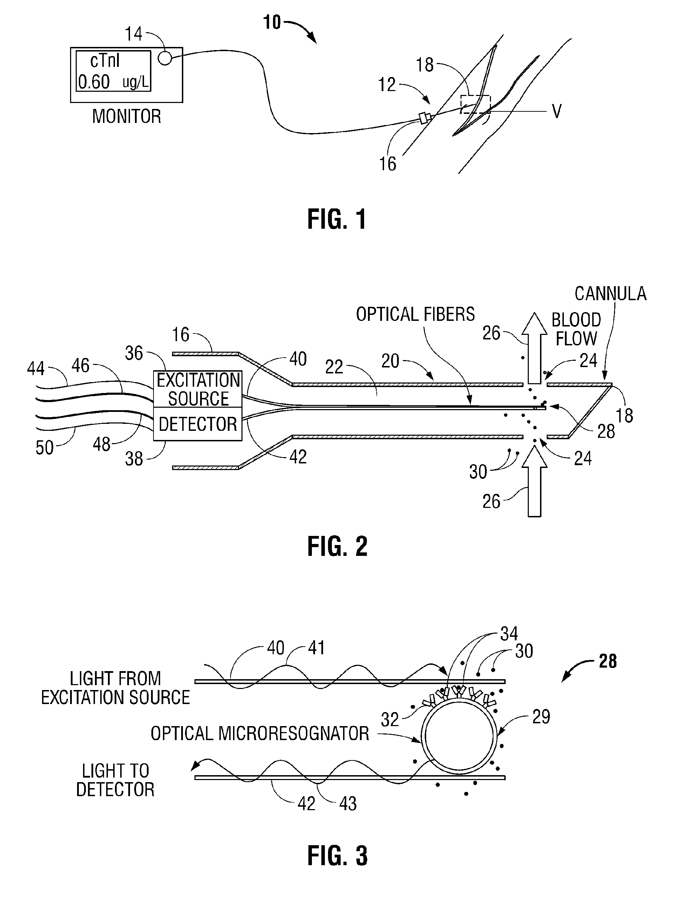

Referring now to Figs. 1-3, blood analyzer 10 in accordance with the principles of the present disclosure is illustrated. Generally, blood analyzer 10 includes an access member or a probe 12 and a monitor 14 in electrical communication with the probe 12. The probe 12 has a proximal end 16 and a distal end 18. The probe 12 may be any tubular structure (e.g., a catheter or a cannula) having a housing 20 and a lumen 22 defined therein and one or more ports 24 at the distal end 18 thereof adapted to provide fluid access to the lumen 22. The distal end 18 of the probe 12 is inserted into a blood vessel "V" to allow for the blood to flow into the lumen as illustrated by directional arrows 26. It is envisioned that the distal end 18 may be configured for penetration and insertion into the blood vessel "V." Alternatively, a tissue-penetrating device may be utilized to create an orifice in the blood vessel "V" into which the probe 12 is later inserted. The blood flows into and through the lumen 22 through the ports 24. Specifically, a portion of the venous circulation is diverted from the vessel "V", passes through the probe 12 and is returned to the venous circulation. An exemplary probe for use with evanescent wave sensors is disclosed in U.S. Patents Nos. 5,340,715 and Nos. 5,156,976, the entire contents of which is incorporated by reference herein.

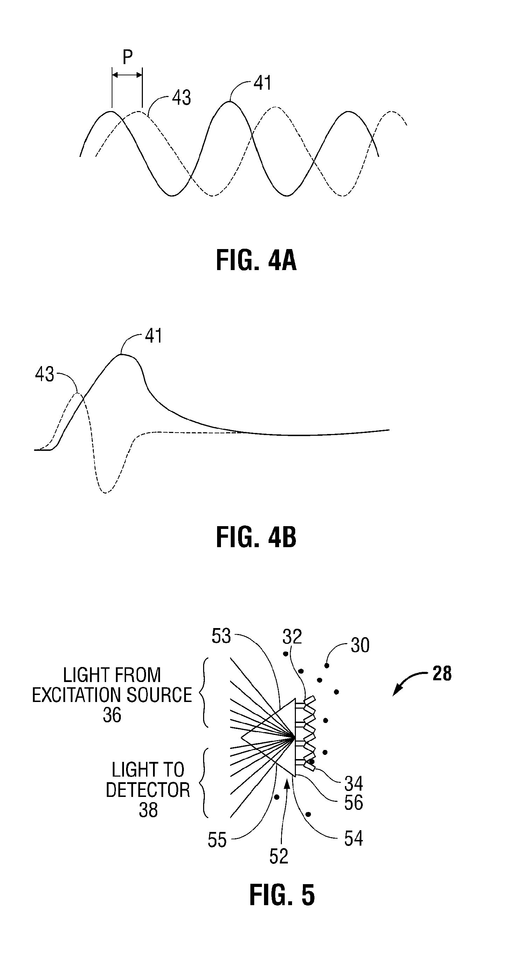

Probe 12 includes an optical biosensor 28 disposed within the lumen 22 which is in fluid communication with the blood flowing through the blood vessel "V." This allows for the blood analyzer 10 to continuously monitor the blood stream for analyte 30 of interest. The biosensor 28 may be an optical sphere-shaped microresonator 29 as shown in Fig. 3 or a prism as shown in Fig. 5. The sphere-shaped microresonator 29 may have a diameter ranging from about 200 to 400 micrometers, preferably about 300 micrometers and may be formed from glass or similar material. The biosensor 28 is configured to resonate in response to excitation light of a predetermined frequency.

The optical microresonator 29 includes a capture agent 32 disposed on the surface thereof. The capture agent 32 may be, for example, specific antibodies adapted to bind to an analyte 30 of interest. Analytes of interest include cardiac troponin, myoglobin, creatinine kinase, creatine kinase isozyme MB, albumin, myeloperoxidase, C-reactive protein, ischemia-modified albumin or fatty acid binding protein and the like. The capture agent 32 may be bound to the surface of the optical microresonator 29 using any number of conventional deposition techniques, such as covalent bonding, physical absorption, or cross-linking to a suitable carrier matrix.

During operation, the microresonator 29 is in fluid communication with the blood. If analyte 30 is present in the blood, the analyte 30 binds to the capture agent 32 to form a bound complex 34. As the capture agent 32 continuously binds to the analyte 30 to form the complex 34, the amount of analyte 30 at or near the surface of the microresonator 29 increases. This, in effect, alters properties of the evanescent field surrounding the microresonator 29 and the light emitted therefrom. The concentration of analyte 30 is capable of being measured by measuring changes in optical properties of the microresonator 29 as discussed hereinbelow.

Prior to commencement of the analysis, the probe 12 is inserted into the blood vessel such that the microresonator 29 is in fluid communication with the blood. The monitor 14 is calibrated. The optical microresonator 29 is optically coupled to an excitation source 36 and a detector 38 via an excitation source waveguide 40 and a detector waveguide 42, respectively. The waveguides 40 and 42 may be optical fibers which are evanescently coupled to the microresonator 29. The optical fibers may be eroded at the distal ends thereof which are coupled to the microresonator 29 by removing reflective cladding from the fibers.

The excitation source 36 supplies an excitation light, shown as an excitation light waveform 41, to the optical microresonator 29 through the excitation source waveguide 40 to excite the biosensor 30 and thereby create an evanescent field around the biosensor 30. The excitation light is supplied to the microresonator 29 at an eigenfrequency, a frequency which induces optical resonance of the microresonator 29. In response to the excitation light, the microresonator 29 emits light, shown as an emitted light waveform 43, which is transmitted through the detector waveguide 42 to the detector 38. Measuring intensity of the emitted light (e.g., light returning from the microresonator 29) allows for the determination of the amount of antigen bound to the microresonator 29.

In a first embodiment, the light supplied by the excitation source 36 is of fixed wavelength and the intensity of the light source is modulated. In this embodiment, the phase lag between the emitted light waveform 43 and the excitation light waveform 41 is solely due to the surface properties of the microresonator 29. This allows for measurement of the phase lag p (e.g., difference) between the emitted light waveform 43 and the excitation light waveform 41 as shown in Fig. 4A. The phase lag is then used to determine the amount of bound antigen.

In an alternate embodiment, the light from the excitation source 36 is pulsed, and the so-called "ring down time," which is a characteristic time constant of the microresonator is measured to characterize the surface properties of the microresonator 29. The ring down time, which is different for each specific analyte, is the time it takes for the intensity of the excitation light waveform 41 to decrease to a predetermined value (e.g., from 100% to 10%) of the emitted light waveform 43 as shown in Fig. 4B. In either embodiment, the detector 38 transmits a signal to the monitor 14 that is indicative of the amount of antigen bound to the microresonator 29.

Throughout this document, the term "continuous" is used to refer to measurements which may be made continuously, or discretely at relatively short time intervals, which are sufficiently brief to result in essentially or approximately continuous measurement. In the aforementioned embodiment , the excitation waveform 41, and therefore the signal to the monitor 14 indicative of the amount of antigen bound to the microresonator, is pulsed rather than continuous. However, in this and similar embodiments, the pulse rate is selected such that the clinically meaningful output to the clinician is essentially continuous. For example, if, for a given pathological condition, the concentration of an analyte of interest is expected to rise to a clinical detection threshold in 1 hour, the collection of one measurement per minute may be sufficient to provide a continuous or approximately continuous clinical measurement.

The excitation source 36 and the detector 38 are coupled to the monitor 14 via two or more wires, excitation wires 44, 46 and emission wires 48, 50, respectively, to the monitor 14. The probe 12 at its proximal end 16 includes a cable 18 which encloses the wires 44, 46, 48, 50. The monitor 14 includes input controls and a display (not explicitly shown). During operation, the excitation source 36 supplies excitation light to the microresonator 29. As analyte 30 binds to the capture agent 32 and complex 34 is formed, the properties of the emitted light waveform change, including changes in intensity, ring down time constant, and phase lag between the excitation light waveform 41 and the emitted light waveform 43. These variables of the emitted light are recorded by the detector 38 which then analyzes the results and determines the amount of the bound analyte.

The detector 38 includes programmable instructions (e.g., algorithm) adapted to calculate the concentration of the analyte 30 as a function of the change in the emitted light. The detector 38 converts the changes in the emitted light measured by the detector 38 and determines presence, concentration and/or change in concentration of the analyte 30. A change in the evanescent field or the emitted light of the microresonator 29 signifies that the analyte 30 has been captured by the capture agent 32 to form the complex 34. The detector 38 transmits the calculations and/or signals corresponding to changes in the evanescent field and/or emitted light of the biosensor 28 to the monitor 14. The monitor 14 then formats the data relating to the concentration of the analyte 30 for output on the display. This step may include displaying that the analyte 30 is present in the blood stream (e.g., displaying text "analyte detected.").

It is further contemplated that the detector 38 is configured to calculate a time derivative of the change in concentration of the analyte 30. In particular, the rate of change of the light properties of the microresonator 29 allows for determination of the concentration of the analyte 30. Taking a second time derivative of the light properties of the microresonator 29 allows for calculation of the rate of change in the concentration of the analyte 30. It is within the purview of those skilled in the art to provide programmable instructions to the detector to enable calculation of derivatives. The data relating to the concentration of the analyte 30 in the bloodstream allows for a more detailed analysis of the test results. In particular, as opposed to simply outputting whether the analyte 30 is present in the bloodstream, knowing the concentration of the analyte 30 and the rate at which the analyte 30 is being generated provides health professionals with a tool to determine the severity of the condition (e.g., myocardial infarction). The detected concentration or the change in concentration of the analyte 30 may be outputted as grams per liter of blood (e.g., µg/L).

The microresonator 29 may operate continuously, wherein the excitation source 36 continuously supplies excitation light to the microresonator 29 and the detector 38 continuously receives the emitted light. In some embodiments, the excitation source 36 pulses the excitation light and the detector 38 measures the so called "ring down time," the characteristic time constant of the microresonator 29 associated with surface properties thereof.

The microresonator 29 ceases to function when all of the antibodies are bound to the analyte 30 and no more analyte 30 can be bound thereto. Therefore, the duration of the functionality of the microresonator 29 varies with the concentration of the analyte 30 in the patient's blood.

Fig. 5 shows another embodiment of the biosensor 28 which employs principles of surface plasmon resonance. In this embodiment, the biosensor 28 includes an optical prism 52 having a first side 53, a base 54 and a second side 55. A metallic layer 56 is disposed on the base 54. The metallic layer 56 is formed from metals such as gold, copper, silver and/or combination thereof. It is also envisioned that a dielectric material may be used as substitute for the metallic layer 56. The metallic layer 56 includes the capture agent 32 disposed on the unattached surface thereof.

The prism 52 is optically coupled to the excitation source 36 and the detector 38. The excitation source 36 supplies a monochromatic light at incident angles through the side 53 sufficient to produce internal reflectance. Due to surface plasmon resonance, the excitation light is partially reflected through the side 55 and partially propagated through the metallic layer 56. The resulting electromagnetic evanescent wave traveling along the metal layer is altered by binding of analyte 30 to the metallic layer 56. The intensity of the reflected light is thereby altered by energy transfer between the evanescent wave and surface plasmons. The excitation source 36 modulates the angle of incidence to identify surface plasmon resonance angle, where the intensity of the reflected light experiences a local minimum.

The detector 38 receives the reflected light from the side 53, measures the intensity of the reflected light and determines the surface plasmon resonance angle. The detector 38 then transmits a signal to the monitor 14 indicative of the amount of analyte 30 bound to the prism 52. The monitor 14 then calculates concentration, rate of change in concentration of the analyte 30.

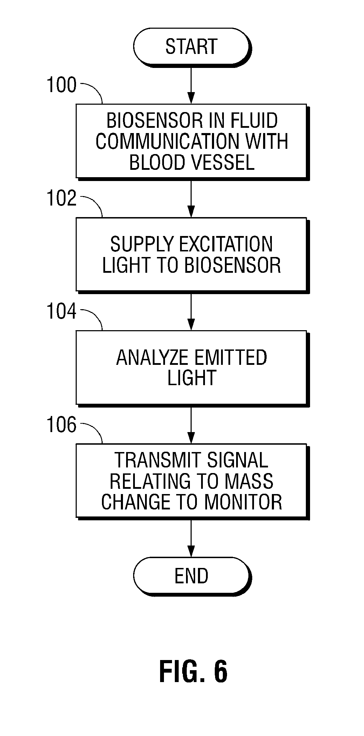

A method for performing a blood assay is illustrated in Fig. 6. In step 100, the biosensor 28 is positioned in fluid communication with the blood. This may be accomplished by positioning the biosensor 28 within an access member (e.g., probe 12) which is then inserted into the blood vessel "V". As discussed above, when the probe 12 is inserted into the blood vessel, the blood flows into the lumen 22 thereby positioning the biosensor 28 in fluid communication with the blood. Alternatively, it is envisioned that blood may be withdrawn through a first lumen of the probe 12 to an external location, passed over the biosensor 28 at the ex vivo location and returned through a second lumen of the probe 12 to the patient.

In step 102, the excitation source 36 transmits excitation light to the biosensor 28, either the microresonator 29 or the prism 52. In step 104, the concentration and change in concentration of the analyte 30 is determined by the detector 38. The detector 38 measures the light returning from the biosensor 28 and determines, based on intensity, ring down time constant, and phase lag of the emitted light, the change in concentration of the analyte 30. As discussed above, the concentration of the analyte 30 is attributed to the binding of the analyte 30 to the capture agent 32 disposed on the surface of the biosensor 28. In particular, the detector 38 calculates the concentration by measuring the optical properties of the biosensor 28. The concentration of the analyte 30 is determined by calculating the rate of change of the optical properties of the biosensor 28. The rate of change of concentration of the analyte 30 is calculated by taking a second time derivative of optical properties of the biosensor 28.

In step 106, the detector 38 transmits the signal relating to the concentration of the analyte 30 to the display of the monitor 14 to provide a clinician with continuous analysis of the level of the analyte 30. The signal may include, but is not limited to, an indicator that analyte 30 is present, an indicator of the concentration of the analyte 30, an indicator of the change in concentration of the analyte 30, and an indicator of the rate of change in concentration of the analyte 30. The clinician then compares the concentration of the analyte to a first predetermined clinical threshold to determine if a particular treatment is warranted.

Further, the monitor 14 is also adapted to display the rate of change in the analyte concentration. The clinician compares the rate of change in analyte concentration to a second predetermined clinical threshold to determine if a particular treatment is warranted. The monitor 14 may optionally include automatic alarms to alert the clinician that the analyte concentration has exceeded one or more threshold values.

While several embodiments of the disclosure have been shown in the drawings and/or discussed herein, it is not intended that the disclosure be limited thereto, as it is intended that the disclosure be as broad in scope as the art will allow and that the specification be read likewise. For example, it is envisioned that the biosensor and/or monitor could evaluate or perform an assay on other body fluid, tissues, enzymes etc. Therefore, the above description should not be construed as limiting, but merely as exemplifications of particular embodiments. Those skilled in the art will envision other modifications within the scope and spirit of the claims appended hereto.

## Claims

1. A medical analyzer to assay blood for an analyte, which comprises:
an optical biosensor adapted to be in fluid communication with a blood vessel whereby blood from the blood vessel contacts the biosensor, the biosensor having at least one material adapted to bind to an analyte of the blood;
a source for supplying excitation light to the biosensor; and
a detector adapted to detect a change in emitted light by the biosensor resulting from the binding of the at least one material of the biosensor with the analyte of the blood and to generate a continuous signal representative of the change in the at least one optical property of the biosensor.

2. The medical analyzer according to claim 1, including a monitor adapted to receive the continuous signal transmitted by the detector and to provide a visual display corresponding to the concentration of the analyte, wherein the detector is further adapted for performing calculations pertaining to the concentration of the analyte.

3. The medical analyzer according to claim 1 or claim 2, including an access member for accessing the blood vessel and having the optical biosensor disposed within a lumen thereof, whereby blood passes through the lumen to contact the biosensor.

4. The medical analyzer according to claim 3, wherein the access member includes a distal end adapted to penetrate the blood vessel to thereby be at least partially positioned therein, the biosensor being disposed within the distal end of the access member.

5. The medical analyzer according to claim 4, wherein the distal end includes an entry port to permit entry of the blood within the lumen of the access member for contacting the biosensor and an exit port for returning the blood to the blood vessel.

6. The medical analyzer according to any preceding claim, wherein the optical biosensor includes an optical sphere-shaped microresonator having the at least one material adapted to bind to the analyte.

7. The medical analyzer according to any preceding claim, wherein the optical biosensor comprises an optical prism having a metallic layer disposed on a base of the optical prism, the metallic layer having the at least one material adapted to bind to the analyte.

8. A method for performing a blood assay, comprising the steps of:
positioning an optical biosensor in fluid communication with a blood vessel whereby blood from the blood vessel contacts the biosensor, the biosensor having at least one material adapted to bind to an analyte;
detecting a change in at least one optical property of the biosensor resulting from binding of the at least one material with the analyte; and
transmitting a continuous signal representative of the change in the at least one optical property of the biosensor to a display module.

9. The method according to claim 8, wherein the optical biosensor comprises an optical microresonator having at least one material bound to the surface thereof and wherein during the step of detecting, the analyte attaches to the at least one material to alter an optical property of the microresonator.

10. The method according to claim 9, wherein the optical microresonator has a substantially spherical shape.

11. The method according to any of claims 8 to 10, wherein the at least one antibody? material is adapted to attach to the analyte, the analyte being selected from the group consisting of cardiac troponin, myoglobin, creatinine kinase, creatine kinase isozyme MB, albumin, myeloperoxidase, C-reactive protein, ischemia-modified albumin and fatty acid binding proteins.

12. The method according to any of claims 8 to 11, wherein the step of positioning includes introducing the biosensor within a lumen of the blood vessel.

13. The method according to claim 12, including the step of accessing the blood vessel with an access member having at least one port to permit passage of the blood therethrough whereby the biosensor is disposed within a distal end of the access member.

14. The method according to any of claims 8 to 13, further including the steps of:
accessing the blood vessel with an access member and withdrawing the blood through the access member to contact the biosensor remote from the blood vessel; and
returning the blood to the blood vessel.

15. The method according to any of claims 8 to 14, wherein the optical property of the biosensor is selected from the group consisting of intensity of emitted light, phase lag and time constant.

16. The method according to claim 8, wherein the optical biosensor comprises an optical prism having a metallic layer disposed on a base of the optical prism, the metallic layer having the at least one material bound to the surface thereof and wherein, during the step of detecting, the analyte attaches to the at least one material.
